# EUROPÄISCHE PATENTSCHRIFT

(11) **EP 4 469 255 B1**
(45) Veröffentlichungstag und Bekanntmachung des Hinweises auf die Patenterteilung: **29.04.2026**
(21) Anmeldenummer: 23700683.8
(22) Anmeldetag: 10.01.2023
(51) Int. Cl.: B29C 45/00, B29C 45/14, B29C 45/17, B29C 65/54, B29C 65/00, A61M 39/12, A61M 39/18, B29K 83/00, B29K 683/00, B29L 23/00

(54) **VERFAHREN ZUR KONNEKTIERUNG ZWEIER SILIKON-SCHLAUCHABSCHNITTE SOWIE PHARMAZEUTISCHES MEDIENTRANSFERVERFAHREN MIT EINEM DERARTIGEN KONNEKTIERUNGSVERFAHREN UND VORRICHTUNG ZUM DURCHFÜHREN DES VERFAHRENS**
METHOD FOR CONNECTING TWO SILICONE TUBE SECTIONS AND PHARMACEUTICAL MEDIA TRANSFER METHOD WITH A CONNECTING METHOD OF THIS TYPE AND DEVICE FOR CARRYING OUT THE METHOD
PROCÉDÉ POUR CONNECTER DEUX PARTIES DE TUYAU EN SILICONE AINSI QUE PROCÉDÉ DE TRANSFERT DE MILIEU PHARMACEUTIQUE COMPORTANT UN TEL PROCÉDÉ DE CONNEXION ET DISPOSITIF POUR LA MISE EN OEUVRE DU PROCÉDÉ

(30) Priorität: 24.01.2022 DE 102022200748
(43) Veröffentlichungstag der Anmeldung: 04.12.2024
(73) Patentinhaber: RAUMEDIC AG, 95213 Münchberg (DE)
(72) Erfinder: GOLLER, Philipp, 95356 Grafengehaig (DE); DRECHSEL, Michael, 95111 Rehau (DE); HAGER, Steffen, 95126 Schwarzenbach an der Saale (OT Martinlamitz) (DE)
(74) Vertreter: Schmitt-Nilson Schraud Waibel Wohlfrom Patentanwälte Partnerschaft mbB
(86) Internationale Anmeldenummer: PCT/EP2023/050449
(87) Internationale Veröffentlichungsnummer: WO 2023/138952

(56) Entgegenhaltungen:
- EP-A1- 3 060 292
- US-A1- 2009 243 284
- US-A1- 2016 200 038
- US-A1- 2021 199 226

## Beschreibung

Die vorliegende Patentanmeldung nimmt die Priorität der deutschen Patentanmeldung DE 10 2022 200 748.4 in Anspruch, deren Inhalt durch Bezugnahme hierin aufgenommen wird.

Die Erfindung betrifft ein Verfahren zur Konnektierung zweier Silikon-Schlauchabschnitte. Ferner betrifft die Erfindung ein pharmazeutisches Medientransferverfahren zur Zuführung eines pharmazeutischen Mediums von einem Ausgangsreservoir hin zu einem Zielreservoir unter Einsatz des Konnektierungsverfahrens, eine Vorrichtung zum Durchführen eines derartigen Verfahrens sowie eine Verwendung eines flüssigen, UV-aushärtenden Silikonmaterials als Umspritzmaterial in einem der genannten Verfahren.

Die Konnektierung von Schlauchabschnitten im Zusammenhang mit pharmazeutischen bzw. biomedizinischen Medientransferverfahren ist im Stand der Technik im Zusammenhang mit Schlauchmaterialien bekannt, die als thermoplastische Elastomere ausgeführt sind. Derartige Verfahren sind bekannt aus der EP 2 637 839 B1, der EP 1 056 970 B 1 und der WO 2021/118780 A1 sowie aus der EP 3 060 292 B1 und der EP 3 603 735 A1. Die EP 2 999 513 B1 offenbart eine Vorrichtung für ein Verfahren zur Herstellung einer sterilen Verbindung von Schläuchen. Die US 2009/0 243 284 A1 offenbart eine Fluidübertragungs-Baugruppe und hierfür zum Einsatz kommende Verfahren. Ein Verfahren umfasst die Schritte: Positionieren einander zugewandter Stirnseiten-Bereiche der zu konnektierenden Silikon-Schlauchabschnitte in einer Umspritzform derart, dass die zugewandten Stirnseiten-Bereiche aneinander liegen, und Umspritzen der aneinander liegenden Stirnseiten-Bereiche in der Umspritzform durch Füllen eines Umspritzhohlraums der Umspritzform mit fließfähigem Silikon. Die DE 10 2019 202 513 A1 offenbart eine Vorrichtung zur Ausbildung oder zum Anspritzen von Kunststoffelementen an Oberflächen eines Halbzeugs.

Außerdem offenbart die US 2021 / 0 199 226 A1 ein Verfahren zur Bereitstellung eines Mehrlumens-Artikels, umfassend: Bereitstellen eines ersten Profils, das mindestens ein Lumen umfasst, wobei das erste Ende und ein erstes Lumen vorhanden sind; Bereitstellen eines zweiten Profils, das ein zweites Ende und ein zweites Lumen umfasst, wobei mindestens das erste Profil, das zweite Profil oder deren Kombination ein polymeres Material umfassen; Bereitstellen einer Oberflächenaktivierungsbehandlung; Behandeln mindestens des ersten Endes, des zweiten Endes oder einer Kombination davon mit der Oberflächenaktivierungsbehandlung; und direktes Kontaktieren des zweiten Endes des zweiten Profils mit dem ersten Ende des ersten Profils, um das erste Ende und das zweite Ende an einer Schnittstelle von erstem Ende und zweitem Ende zusammenzuführen und eine Fluidleitung bereitzustellen, wobei das erste Lumen einen Flüssigkeitsfluss in einem ersten Pfad und das zweite Lumen einen Flüssigkeitsfluss in einem von dem ersten Pfad unterschiedlichen Pfad aufweist.

Die US 2016 / 0 200 038 A1 offenbart ein Verfahren zur Herstellung einer Flüssigkeitsmanagement-Anordnung, die Folgendes umfasst: Koppeln eines ersten und eines zweiten Silikonschlauchabschnitts an einen Verbinder aus Kunststoffmaterial, der mindestens einen Einlass und mindestens einen Auslass sowie ein dazwischen verlaufendes Lumen aufweist, wobei der mindestens eine Einlass an einem ersten Übergang mit dem ersten Silikonschlauchabschnitt verbunden ist und der mindestens eine Auslass an einem zweiten Übergang mit dem zweiten Silikon-schlauchabschnitt verbunden ist; Einführen des Verbinders in eine Kavität einer Form; Einspritzen von flüssigem Silikonkautschuk in die Formkavität; und Anwenden von UV-Strahlung auf den flüssigen Silikonkautschuk, um das Silikon auszuhärten.

Es ist eine Aufgabe der vorliegenden Erfindung, ein Verfahren zur Konnektierung zweier Silikon-Schlauchabschnitte so weiterzubilden, dass es sicher, reproduzierbar und automatisiert mit vertretbarem Aufwand durchgeführt werden kann.

Diese Aufgabe ist erfindungsgemäß gelöst durch ein Konnektierungsverfahren mit den im Anspruch 1 angegebenen Merkmalen.

Erfindungsgemäß wurde erkannt, dass ein Umspritzen aneinander liegender Stimseiten-Bereiche der zu konnektierenden Silikon-Schlauchabschnitte mit fließfähigem Silikon, welches nachfolgend ausgehärtet wird, eine sichere Konnektierung slösung ermöglicht. Soweit entsprechend sterilisierte Silikon-Schlauchabschnitte konnektiert werden, ergibt sich eine bioverträgliche und sichere Fluidverbindung zwischen den konnektierten Silikon-Schlauchabschnitten.

Bei dem fließfähigen Silikon kann es sich um Liquid Silicone Rubber (LSR, Flüssig-Silikon) handeln. Es kann sich um eine LSR-Materialtype im Härtebereich zwischen Shore A20 bis Shore A70 handeln.

Die Fließfähigkeit des Silikons ist derart, dass der Umspritzhohlraum ausreichend gefüllt wird. Die Viskosität des fließfähigen Silikons kann dabei an eine typische Kavitätsgröße bzw. Kavitätsgestaltung des Umspritzhohlraums angepasst sein.

Ein Silikon-Schlauchabschnitt im Sinne der vorliegenden Anmeldung ist ein Schlauchabsclmitt aus einem Material, das überwiegend aus Silikon besteht. Die aneinander liegenden Stimseiten-Bereiche der Silikon-Schlauchabschnitte müssen nicht über einen gesamten Schlauchumfang aneinander anliegen. Dies ist allerdings bevorzugt.

Das Aushärten des fließfähigen Silikons erfolgt mithilfe einer UV-Licht-Bestrahlung, insbesondere im UV-A Wellenlängenbereich im Bereich zwischen 315 nm und 400 nm, besonders im Bereich zwischen 315 nm und 380 nm. Grundsätzlich kann ein Aushärten des fließfähigen Silikons mittels UV-Lichtbestrahlung auch durch Bestrahlen mit einer anderen UV-Wellenlänge im Bereich zwischen 100 nm und 315 nm erfolgen, insbesondere im Bereich zwischen 280 nm und 315 nm (UV-B). Es kann dabei eine UV-Licht breitbandig emittierende Lichtquelle zum Einsatz kommen, bei der ein tatsächlich verwendeter UV-Wellenlängenbereich dann herausgefiltert wird.

Eine weitere Aufgabe der Erfindung ist es, ein pharmazeutisches Medientransferverfahren zur Zuführung eines pharmazeutischen Mediums von einem Ausgangsreservoir hin zu einem Zielreservoir hinsichtlich seiner Betriebs- und Handling-Sicherheit zu verbessern.

Diese Aufgabe ist erfindungsgemäß gelöst durch ein Medientransferverfahren mit den in Anspruch 2 angegebenen Merkmalen. Durch den Einsatz des erfindungsgemäßen Konnektierungsverfahrens im pharmazeutischen Medientransferverfahren kommen dessen Vorteile besonders gut zum Tragen. Ein derartiger Medientransfer kann in einer gesamten Produktionskette eines pharmazeutischen Herstellungsprozesses stattfinden, beispielsweise im Rahmen des Herstellungsprozesses von einem Batchansatz bis zu einem finalen Abfüllprozess. Das Medientransferverfahren kann im Rahmen von Pharmazeutika-Entwicklungsschritten von der Laborentwicklung über Scale-Up-Zwischenschritte bis hin zu einer Massenproduktion zum Einsatz kommen. Ein Beispiel für das pharmazeutische Medientransferverfahren ist ein pharmazeutisches Abfüllverfahren, insbesondere das Zuführen einer Pufferlösung zur pH-Wert-Regulierung im Zielreservoir. Als Zielreservoir kann ein steriles Behältnis, ein Prozessreaktor oder auch ein Beutel zum Einsatz kommen.

Zum pharmazeutischen Medientransferverfahren gehört auch ein Sterilisationsschritt für die Stimseiten-Bereiche der beim Schneiden erzeugten Schlauchabschnitte. Ein derartiges Sterilisieren der Stimseiten-Bereiche erfolgt mittels einer UV-Licht-Bestrahlung. Hierbei kann eine UV-C Wellenlänge im Wellenlängenbereich zwischen 100 nm und 280 nm, besonders im Bereich zwischen 100 nm und 200 nm oder zwischen 200 nm und 280 nm zum Einsatz kommen.

Ein Durchschneiden nach Anspruch 3 hat sich als besonders geeignet herausgestellt. Das Durchschneiden kann rein mechanisch als Kaltschnitt, also ohne Temperatur-Wirkung, erfolgen. Die Schneidetemperatur kann kleiner sein als 70°C, kann kleiner sein als 60°C, kann kleiner sein als 50°C, kann kleiner sein als 40°C und kann auch kleiner sein als 30°C. Die Schneidetemperatur ist regelmäßig größer als 10°C.

Die Vorteile einer Vorrichtung nach Anspruch 4 entsprechen denen, die vorstehend in Bezug auf die Verfahren bereits erläutert wurden. Als Positioniereinrichtung kann eine Einrichtung zum Einsatz kommen, die zum Positionieren im Zusammenhang mit dem Konnektieren von TPE-Schlauchkomponenten bereits bekannt ist.

Eine mobile Ausführung nach Anspruch 7 ist besonders geeignet für den Labor- bzw. Klinikeinsatz. Auch in einer Reinraum- oder in einer Vorserienumgebung kann diese mobile Ausführung vorteilhaft zum Einsatz kommen. Die Konnektierungsvorrichtung kann fahrbar gestaltet sein. Die das Konnektierungsverfahren durchführende Vorrichtung kann insbesondere fahrbar gestaltet sein. Hierzu kann die Vorrichtung mehrere Fahr-Rollen aufweisen, die an einem Rahmen der Vorrichtung gelagert sind. Die Vorrichtung kann bei der fahrbaren Ausgestaltung auch einen Fahrantrieb aufweisen, beispielsweise über mindestens einen Elektromotor. Die Konnektierungsvorrichtung kann eine autarke Energieversorgung haben, kann also so ausgelegt sein, dass sie nicht auf externe Netzanschlüsse angewiesen ist. Die Konnektierungsvorrichtung kann für einen Batteriebetrieb ausgeführt sein.

Die Konnektierungsvorrichtung kann ein Set von Schneideinheiten, zum Beispiel von Messern aufweisen. Die Konnektierungsvorrichtung kann ein Set von Wechsel-Umspritzformen aufweisen. Die Konnektierungsvorrichtung kann eine Leseeinheit zur Vorgabe eines Messers innerhalb des Messer-Sets und/oder zur Vorgabe einer Umspritzform innerhalb des Umspritzform-Sets aufweisen. Diese Vorgabe kann jeweils abgestimmt auf die zu konnektierenden Schlauchabschnitte erfolgen. Zur entsprechenden Vorgabe können die Schläuche für die zu konnektierenden Schlauchabschnitte Vorgabedaten aufweisen, die kodiert zum Beispiel in Form eines QR-Codes vorliegen können. Die Leseeinheit kann dann als QR-Code-Leser ausgeführt sein.

Eine Verwendung eines konnektierten Silikon- Schlauchs führt zu Vorteilen, die vorstehend bereits diskutiert sind. Es kann sich insbesondere eine robuste und reißfeste Konnektierung ergeben, die gleichzeitig einen sicheren Medien- bzw. Fluiddurchgang gewährt.

Die Verwendung eines UV-aushärtenden Silikonmaterials nach Anspruch 10 hat sich als fürs Konnektierungsverfahren besonders geeignet herausgestellt.

Bei dem fließfähigen Silikonmaterial kann es sich um ein 1K- oder um ein 2K-Material handeln.

Ein Härtebereich des Silikonmaterials nach Anspruch 11 hat sich zur Herstellung einer sicheren und gleichzeitig stabilen Verbindung als besonders geeignet herausgestellt.

Unterschiedliche UV-Wellenlängen zum Aushärten einerseits und zum Sterilisieren andererseits nach Anspruch 1, 4 und 10 haben sich als für die Prozesssicherheit sowie für die Prozessgeschwindigkeit besonders geeignet herausgestellt. Bei der UV- Aushärte Wellenlänge kann es sich um eine UV-A Wellenlänge handeln. Bei der UV-Sterilisierungswellenlänge kann es sich um eine UV-C Wellenlänge handeln.

Ausführungsbeispiele der Erfindung werden nachfolgend anhand der Zeichnung näher erläutert.

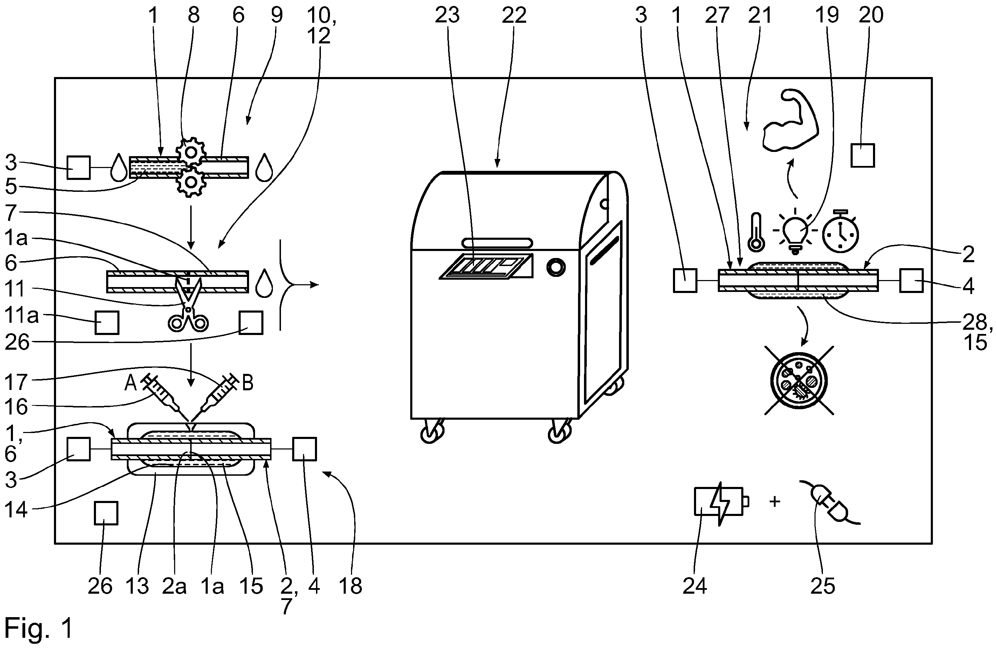

Die einzige Figur 1 zeigt ein Ablaufschema eines Verfahrens zur Konnektierung zweier Silikon-Schlauchabschnitte sowie eine Vorrichtung zur Durchführung des Verfahrens.

Ein Verfahren zur Konnektierung zweier Silikon-Schlauchabschnitte 1, 2 kommt bei einem pharmazeutischen Medientransferverfahren, insbesondere bei einem pharmazeutischen Abführverfahren zur Zuführung eines pharmazeutischen Mediums von einem Ausgangsreservoir 3 hin zu einem Zielreservoir 4 zum Einsatz. Prinzipiell kann das Konnektierungsverfahren auch bei einem anderen pharmazeutischen, biologischen, medizintechnischen oder auch sonstigen naturwissenschaftlichen oder industriellen Verfahren zum Einsatz kommen.

Bei den Silikon-Schlauchabschnitten 1, 2 handelt es sich um Schlauchabschnitte aus einem Material, das überwiegend, also zu mehr als 50 Gewichtsprozent, aus Silikon besteht. Dieses Silikonmaterial der Silikon-Schlauchabschnitte 1, 2 kann auch zu mehr als 60 Gewichtsprozent, zu mehr als 70 Gewichtsprozent, zu mehr als 75 Gewichtsprozent, zu mehr als 80 Gewichtsprozent, zu mehr als 85 Gewichtsprozent, zu mehr als 90 Gewichtsprozent, zu mehr als 95 Gewichtsprozent, oder auch zu mehr als 98 Gewichtsprozent aus Silikon bestehen.

Das Ausgangsreservoir 3 steht dabei mit dem Silikon-Schlauchabschnitt 1 und das Zielreservoir 4 steht mit dem Silikon-Schlauchabschnitt 2 in Medienverbindung, zum Beispiel in Fluidverbindung, insbesondere zur Führung eines flüssigen Mediums.

Das pharmazeutische Abfüllverfahren läuft folgendermaßen ab:
Zunächst wird ein pharmazeutisches Medium 5 im Ausgangsreservoir 3 einschließlich eines mit dem Ausgangsreservoir 3 in Medienverbindung stehenden Silikon-Zuführschlauchs 6 bereitgestellt. Der Silikon-Schlauchabschnitt 1 ist Teil des Silikon-Zuführschlauchs 6. Zudem wird das Zielreservoir 4 einschließlich eines mit diesem in Medienverbindung stehenden Silikon-Abführschlauchs 7 bereitgestellt. Der Silikon-Schlauchabschnitt 2 ist Teil des Silikon-Abführschlauchs 7.

Nun wird das pharmazeutische Medium 5 in einem Silikon-Zuführschlauchabschnitt des Silikon-Zuführschlauchs 6 insbesondere hin zum Ausgangsreservoir 3 verdrängt, was in der Figur 1 links oben angedeutet ist, wo das pharmazeutische Medium 5 mithilfe eines Verdrängungsmechanismus 8 aus einer rechten Hälfte des dargestellten Abschnitts des Silikon-Zuführschlauchs 6 verdrängt wird. Figur 1 zeigt links oben also einen Verdrängungsschritt 9 des Abfüllverfahrens.

In einem Schneideschritt 10 (vgl. Fig. 1 links Mitte) des Abfüllverfahrens wird dann der Silikon-Zuführschlauchabschnitt des Silikon-Zuführschlauchs 6 mit einer Schneideinheit 11 zur Erzeugung eines sterilen Stirnseiten-Bereichs 1a des Silikon-Zuführschlauchabschnitts durchgeschnitten. In gleicher Weise wird in einem weiteren Schneideschritt 12, der ebenfalls in der Figur 1 links Mitte veranschaulicht ist, ein Silikon-Abführschlauchabschnitt des Silikon-Abführschlauchs 7 zur Erzeugung eines sterilen Stirnseiten-Bereichs 2a des Silikon-Abführschlauchabschnitts mit der Schneideinheit 11 durchgeschnitten. Bei der Schneideinheit 11 kann es sich um ein wechselbares Messer handeln.

Die beiden Schneideschritte 10, 12 zum Durchschneiden der Schläuche 6, 7 können zeitgleich mit der gleichen Schneideinheit 11 erfolgen. Hierzu können die beiden Schläuche 6, 7 parallel zueinander angeordnet sein.

Die beiden Schlauchabschnitte, als einerseits der Silikon-Zuführschlauchabschnitt und andererseits der Silikon-Abführschlauchabschnitt, stellen nach den Schneideschritten 10, 12 die beiden zu konnektierenden Silikon-Schlauchabschnitte 1, 2 dar.

Das Durchschneiden des Silikon-Zuführschlauchabschnitts 6 und des Silikon-Abführschlauchabschnitts 7 erfolgt mit der Schneideeinheit 11 bei einer Schneidetemperatur, die kleiner ist als 80°C. Diese Temperatur kann kleiner sein als 70°C, kann kleiner sein als 60°C, kann kleiner sein als 50°C, kann kleiner sein als 40°C, kann kleiner sein als 30°C. Es handelt sich bei den Schneideschritten 10, 12 jeweils um einen Kaltschnitt, der rein mechanisch, also nicht unter Hitzeeinwirkung auf das Silikonmaterial der Silikon-Schlauchabschnitte erfolgt.

Ein Sterilisieren der beiden Stirnseiten-Bereiche 1a, 2a kann im Rahmen des Abfüllverfahrens mithilfe einer UV-Sterilisierungsbeleuchtung dieser Stirnseiten-Bereiche 1a, 2a erfolgen.

Nach den Schneideschritten 10, 12 werden die beiden Silikon-Schlauchabschnitte 1, 2 mit den Stirnseiten-Bereichen 1a, 2a einander zugewandt in einer Umspritzform 13 so zueinander positioniert, dass die zugewandten Stirnseiten-Bereiche 1a, 2a aneinander anliegen. Zum Positionieren der zu konnektierenden Silikon-Schlauchabschnitte 1, 2 kann eine Positioniereinrichtung dienen, die grundsätzlich aus dem Stand der Technik bereits bekannt ist. Eine derartige Positioniereinrichtung ist in der Figur 1 links Mitte bei 11a schematisch angedeutet.

Nun werden die aneinander liegenden Stirnseiten-Bereiche 1a, 2a in der Umspritzform 13 durch Füllen eines Umspritzhohlraums 14 der Umspritzform 13 mit fließfähigem Silikon 15 umspritzt.

Bei dem fließfähigen Silikon kann es sich um ein Zweikomponenten (2K)-Material mit Komponenten A und B handeln, die über getrennte Zuführ-Dosierkanäle mit Dosiereinheiten 16, 17 dem Umspritzhohlraum 14 zugeführt werden. Alternativ kann es sich bei dem fließfähigen Silikon auch um ein Einkomponenten (1K)-Material handeln. Soweit es sich bei dem fließfähigen Silikon 15 um ein einkomponentiges Material handelt, sind in dieser einen Komponente ein Grundpolymer, ein Katalysator und ein Vernetzer enthalten. Soweit es sich bei dem fließfähigen Silikon 15 um ein zweikomponentiges Material handelt, kann in einer der beiden Komponenten ein Grundpolymer und ein Vernetzer und in der weiteren Komponente ein Katalysator vorliegen.

Die Dosiereinheiten 16, 17 stellen eine Zuführeinrichtung für das fließfähige Silikon 15 als Umspritzmaterial dar.

Es wird ein fließfähiges, UV-aushärtendes Silikonmaterial in Form des fließfähigen Silikons 15 als Umspritzmaterial im pharmazeutischen Abfüllverfahren, insbesondere im Konnektierungsverfahren, verwendet.

Die Figur 1 zeigt links unten einen Umspritzschritt 18 des Konnektierverfahrens nach dem vorausgegangenen und nicht im Einzelnen dargestellten Positionierschritt.

Nach dem Umspritzen 18 erfolgt ein Aushärten des fließfähigen Silikons durch Bestrahlen des fließfähigen Silikons 15 über eine UV-Lampe 19 mit einer UV-Aushärtewellenlänge. Die UV-Aushärtewellenlänge kann sich von der UV-Sterilisierungswellenlänge unterscheiden. Das aus dem fließfähigen Silikon 15 hervorgehende ausgehärtete Silikonmaterial kann eine Härte im Bereich Shore A 40 bis Shore A 60 haben.

Eine Aushärtebestrahlung und eine Sterilisierungsbestrahlung können im Zuge des pharmazeutischen Abfüllverfahrens im gleichen Prozessschritt erfolgen.

Eine Bestrahlungsdauer beim Aushärten bzw. Vernetzen des fließfähigen Silikons 15 liegt im Bereich zwischen 10 s und 120 s, zum Beispiel zwischen 60 s und 120 s. Eine Vernetzungswellenlänge liegt im UV-A Bereich, also in einem Wellenlängenbereich zwischen 315 nm und 400 nm.

Beim Sterilisieren liegt eine Bestrahlungsdauer im Bereich zwischen 5 s und 1 min, zum Beispiel zwischen 10 s und 50 s oder zwischen 15 s und 45 s. Eine Sterilisierungs-Bestrahlungs-Wellenlänge liegt im UV-C Bereich, also in einem Wellenlängenbereich zwischen 200 nm und 280 nm, beispielsweise in einem Bereich zwischen 240 nm und 260 nm.

Für die Vernetzungs-Bestrahlung einerseits und für die Sterilisierungs-Bestrahlung andererseits können zwei unterschiedliche UV-Quellen zum Einsatz kommen. Alternativ ist es möglich, mit einer gemeinsamen UV-Quelle zu arbeiten, bei der dann über entsprechende Filter die jeweiligen Bestrahlungswellenlängen zum Vernetzen/Aushärten sowie zum Sterilisieren vorgegeben werden.

Die Vernetzungs-Bestrahlungsdauer kann länger sein als die Sterilisations-Bestrahlungsdauer. Alternativ kann auch die Sterilisations-Bestrahlungsdauer länger sein als die Vernetzungs-Bestrahlungsdauer. Beide Bestrahlungsdauern können auch gleich lang sein.

Die Vernetzungs-/Aushärte-Bestrahlung erfolgt zeitlich nicht überlappend mit der Sterilisierungs-Bestrahlungsdauer. Erfindungsgemäß erfolgt die Sterilisierungs-Bestrahlung im Ablauf des Konnektierungsverfahrens vor der Vernetzungs-/Aushärte-Bestrahlung.

Die Sterilisier- bzw. Aushärtebestrahlung erfolgt über eine Steuer-Regeleinheit 20 temperatur- und/oder zeitgesteuert bzw. -geregelt. Ein entsprechender Aushärteschritt 21 ist in der Figur 1 rechts oben dargestellt. Figur 1 Mitte zeigt eine Konnektierungsvorrichtung 22 zur Durchführung des pharmazeutischen Abfüllverfahrens und insbesondere zur Durchführung des Konnektierungsverfahrens.

Zur Konnektierungsvorrichtung 22 gehören das wechselbare Messer 11 sowie die Umspritzform 13. Die Konnektierungsvorrichtung 22 kann ein Magazin mit mehreren wechselbaren Messern 11 bzw. Wechselklingen aufweisen, die wahlweise und insbesondere automatisiert zum Einsatz kommen können. Mit einer Klinge können beispielsweise zehn bis hundert Schneidvorgänge durchgeführt werden. Die Konnektierungsvorrichtung 22 kann ein Set von Messern 11 aufweisen. Die jeweilige Umspritzform 13 kann also auf die zu konnektierenden Schlauchabschnitte 1, 2 abgestimmt sein.

Die Konnektierungsvorrichtung 22 kann ein Set von Umspritzformen 13 insbesondere zur Aufnahme verschiedener Außendurchmesser von Silikon-Schlauchabschnitten nach Art der Silikon-Schlauchabschnitte 1, 2 aufweisen.

Die Umspritzform 13 kann als Wechsel-Umspritzform gestaltet sein. Die Konnektierungsvorrichtung 22 kann ein Formmagazin aufweisen, in dem eine Mehrzahl derartiger Umspritz-Formen, insbesondere auch mit mehreren, wählbaren Größen des Umspritzhohlraums aufweisen. Ein Wechsel zwischen den Wechsel-Umspritzformen kann automatisiert erfolgen. Pro Umspritzform können beispielsweise 500 bis 100.000, insbesondere 10.000 bis 25.000 Umspritzvorgänge durchgeführt werden. Die Konnektierungsvorrichtung 22 kann zwischen 3 und 12 Umspritzformen-Größen bevorraten, die insbesondere auf Pharma-Standardgrößen der Außendurchmesser der Silikon-Schlauchabschnitte 1, 2 abgestimmt sind.

Die Umspritzform 13 kann in einer Formaufnahme der Konnektierungsvorrichtung 22 aufgenommen sein, die eine Kontur hat, die komplementär ist zur Kontur der aufgenommenen Umspritzform. Hierüber kann zum einen eine Verdrehsicherung der Umspritzform in der Formaufnahme gewährleistet sein und zum anderen gewährleistet werden, dass die Umspritzform in der Formaufnahme korrekt orientiert bzw. positioniert ist.

Zwischen der Umspritzform und der Formaufnahme kann eine Signalverbindung eingerichtet sein, die gewährleistet, dass für eine aktuelle Konnektierungsaufgabe die korrekte Umspritzform eingesetzt ist. Dies kann beispielsweise durch eine Mehrzahl von Kontaktstiften der Formaufnahme erreicht werden, zwischen denen über die Umspritzform bei korrekter Auswahl entsprechende leitende Verbindungen hergestellt werden.

Die Umspritzform 13 kann aus einem Polymermaterial gefertigt sein, beispielsweise aus PMMA.

Eine Betriebsdauer und eine Anzahl von Verwendungszyklen des jeweiligen mittelbaren Messers 11 können in der Steuer-/Regeleinheit 20, die auch zur Ansteuerung des Messers 11 dient, vorgegeben und dokumentiert werden.

Die Steuer-/Regeleinheit 20 dient zudem zur Dokumentation des Betriebs der jeweiligen UV-Lampe einerseits zum Aushärten und andererseits zum Sterilisieren. Jeder Konnektierungsvorgang kann dabei in der Steuer-/Regeleinheit 20 ausgewertet werden.

Insbesondere eine Strahlungsintensität, eine Bestrahlungsdauer sowie eine Temperatur insbesondere des fließfähigen Silikons 15 beim Aushärtevorgang bzw. der Stirnseiten-Bereiche 1a, 2a beim Sterilisierungsvorgang können dabei vorgegeben und dokumentiert werden.

Nach erfolgtem Konnektieren findet ein Abfüllen des pharmazeutischen Mediums vom Ausgangsreservoir 3 über die nun miteinander konnektierten Silikon-Schlauchabschnitte 1, 2 hin zum Zielreservoir 4 statt. Bei dem pharmazeutischen Medium handelt es sich beispielsweise um eine Pufferlösung zur pH-Wert-Regulierung.

Die Konnektierungsvorrichtung 22 kann mobil ausgeführt sein. Die Konnektierungsvorrichtung 22 hat eine Anzeige/Bedieneinheit 23, die als Touchscreen ausgeführt sein kann und mit der Steuer-/Regeleinheit 20 in Signalverbindung steht.

Die Konnektierungsvorrichtung 22 kann fahrbar ausgestaltet sein. Die Konnektierungsvorrichtung 22 kann für einen Batteriebetrieb 24 und/oder für einen Netzbetrieb 25 ausgeführt sein.

Die Konnektierungsvorrichtung 22 kann eine Leseeinheit 26 zur Vorgabe des jeweiligen Messers 11 und/oder zur Vorgabe der jeweiligen Umspritzform 13 aufweisen, was in den Figuren 1 links Mitte und 1 links unten jeweils schematisch dargestellt ist. Die Leseeinheit 26 kann dabei einen Code auf dem jeweiligen Messer 11 und/oder auf der jeweiligen Umspritzform 13 lesen, beispielsweise einen QR-Code. Alternativ oder zusätzlich kann die Leseeinheit 26 einen entsprechenden Code auf wenigstens einen der Silikon-Schlauchabschnitte 1, 2 lesen, der wiederum als QR-Code ausgeführt sein kann.

Ein fertig konnektierter Silikon-Schlauch 27 mit den beiden konnektierten, sterilen Stirnseiten-Bereichen 1a, 2a der Silikon-Schlauchabschnitte 1, 2, die über eine Umspritzung 28 miteinander verbunden sind, wird zur Medienzuführung in einem pharmazeutischen Abfüllprozess verwendet.

## Patentansprüche

1. Verfahren zur Konnektierung zweier Silikon-Schlauchabschnitte (1, 2) mit folgenden Schritten:
Sterilisieren der Stirnseiten-Bereiche (1a, 2a) der zu konnektierenden Silikon-Schlauchabschnitte (1, 2) mittels einer UV-Licht-Bestrahlung mit einer UV-Sterilisierungswellenlänge;
Positionieren der einander zugewandten Stirnseiten-Bereiche (1a, 2a) der zu konnektierenden Silikon-Schlauchabschnitte (1, 2) in einer Umspritzform (13) derart, dass die zugewandten Stirnseiten-Bereiche (1a, 2a) aneinander liegen;
Umspritzen der aneinander liegenden Stirnseiten-Bereiche (1a, 2a) in der Umspritzform (13) durch Füllen eines Umspritzhohlraums (14) der Umspritzform (13) mit einem fließfähigem, UV-aushärtenden Silikon (15);
Aushärten (21) des fließfähigen Silikons (15) durch UV- Lichtbestrahlung mittels einer UV-Licht-Bestrahlung mit einer UV-Aushärtewellenlänge;
wobei sich die UV-Aushärtewellenlänge von der UV-Sterilisierungswellenlänge unterscheidet.

2. Pharmazeutisches Medientransferverfahren zur Zuführung eines pharmazeutischen Mediums (5) von einem Ausgangsreservoir (3) hin zu einem Zielreservoir (4) mit folgenden Schritten:
Bereitstellen des pharmazeutischen Mediums (5) im Ausgangsreservoir (3) einschließlich eines mit dem Ausgangsreservoir (3) in Medienverbindung stehenden Silikon-Zuführschlauchs (6);
Bereitstellen des Zielreservoirs (4) einschließlich eines mit dem Zielreservoir (4) in Medienverbindung stehenden Silikon-Abführschlauchs (7);
Verdrängen (9) des pharmazeutischen Mediums (5) in einem Silikon-Zuführschlauchabschnitt des Silikon-Zuführschlauchs (6);
Durchschneiden (10) des Silikon-Zuführschlauchabschnitts des Silikon-Zuführschlauchs (6) zur Erzeugung eines sterilen Stirnseiten-Bereichs (1a) des Silikon-Zuführschlauchabschnitts;
Durchschneiden eines Silikon-Abführschlauchabschnitts des Silikon-Abführschlauchs (7) zur Erzeugung eines sterilen Stirnseiten-Bereichs (2a) des Silikon-Abführschlauchabschnitts;
Konnektieren des Silikon-Zuführschlauchabschnitts mit dem Silikon-Abführschlauchabschnitts mit einem Verfahren nach Anspruch 1, wobei der Silikon-Zuführschlauchabschnitt einerseits und der Silikon-Abführschlauchabschnitt andererseits die beiden mit dem Verfahren zu konnektierenden Silikon-Schlauchabschnitte darstellen;
Überführen des pharmazeutischen Mediums (5) vom Ausgangsreservoir (3) hin zum Zielreservoir (4) über die konnektierten Schlauchabschnitte (1, 2).

3. Pharmazeutisches Medientransferverfahren nach Anspruch 2, **dadurch gekennzeichnet, dass** das Durchschneiden (10) des Silikon-Zuführschlauchabschnitts und/oder des Silikon-Abführschlauchabschnitts bei einer Schneidetemperatur erfolgt, die kleiner ist als 80°C.

4. Vorrichtung (22) zur Konnektierung zweier Silikon-Schlauchabschnitte (1, 2) und zur Durchführung eines Verfahrens nach einem der Ansprüche 1 bis 3
mit einer UV-Quelle zum Sterilisieren der Stirnseiten-Bereiche (1a, 2a) der zu konnektierenden Silikon-Schlauchabschnitte (1, 2) mittels einer UV-Licht-Bestrahlung mit einer UV-Sterilisierungswellenlänge;
mit einer Schneideinheit (11) zum Durchschneiden mindestens eines der Silikon-Schlauchabschnitte (1, 2),
mit einer Umspritzform (13) einschließlich einer Zuführeinrichtung (16, 17) für das fließfähige Silikon (15) als Umspritzmaterial,
mit einer Positioniereinrichtung (11a) zum Positionieren der zu konnektierenden Silikon-Schlauchabschnitte (1, 2); und
mit einer UV-Quelle zum Aushärten des fließfähigen Silikons (15) durch UV-Lichtbestrahlung mittels einer UV-Licht-Bestrahlung mit einer UV-Aushärtewellenlänge;
wobei sich die UV-Aushärtewellenlänge von der UV-Sterilisierungswellenlänge unterscheidet.

5. Vorrichtung nach Anspruch 4,
wobei die Vorrichtung eine erste UV-Quelle zum Sterilisieren der Stirnseiten-Bereiche (1a, 2a) der zu konnektierenden Silikon-Schlauchabschnitte (1, 2) mittels einer UV-Licht-Bestrahlung mit einer UV-Sterilisierungswellenlänge und eine zweite UV-Quelle zum Aushärten des fließfähigen Silikons (15) mittels einer UV-Licht-Bestrahlung mit einer UV-Aushärtewellenlänge aufweist.

6. Vorrichtung nach Anspruch 4,
wobei die Vorrichtung eine gemeinsame UV-Quelle aufweist, die so ausgebildet ist, dass über entsprechende Filter die jeweiligen Bestrahlungswellenlängen zum Sterilisieren der Stirnseiten-Bereiche (1a, 2a) der zu konnektierenden Silikon-Schlauchabschnitte (1, 2) und zum Aushärten des fließfähigen Silikons (15) vorgebbar sind.

7. Vorrichtung nach einem der Ansprüche 4 bis 6, **gekennzeichnet durch** eine mobile Ausführung für den Laboreinsatz, den Klinikeinsatz, den Einsatz in einer Reinraumumgebung oder für den Einsatz in einer Vorserienumgebung;
wobei die Vorrichtung fahrbar ausgebildet ist und mehrere Fahr-Rollen aufweist, die an einem Rahmen der Vorrichtung gelagert sind;
wobei die Vorrichtung insbesondere einen Fahrantrieb, insbesondere einen Elektromotor aufweist.

8. Vorrichtung nach einem der Ansprüche 4 bis 7, weiterhin aufweisend
ein Set von Messern (11); und
ein Set von Umspritzformen (13);
wobei die Schläuche für die zu konnektierenden Schlauchabschnitte, nämlich der Silikon-Zuführschlauch (6) und der Silikon-Abführschlauch (7), Vorgabedaten aufweisen;
wobei die Vorrichtung weiterhin eine Leseeinheit (26) aufweist, zur Vorgabe eines Messers innerhalb des Sets von Messern (11) und/oder zur Vorgabe einer Umspritzform innerhalb des Sets von Umspritzformen (13), jeweils abgestimmt auf die zu konnektierenden Schlauchabschnitte.

9. Vorrichtung nach Anspruch 8,
wobei die Messer (11) des Sets von Messern (11) und die Umspritzformen (13) des Sets von Umspritzformen (13) jeweils eine Code, insbesondere einen QR-Code aufweisen;
wobei die Vorgabedaten der Schläuche (6, 7) für die zu konnektierenden Schlauchabschnitte in Form von Codes, insbesondere QR-Codes vorliegen; und
wobei die Leseeinheit (26) als Code-Leser, insbesondere als QR-Code-Leser ausgeführt ist.

10. Verwendung eines flüssigen, UV-aushärtenden Silikonmaterials (15) als Umspritzmaterial in einem Verfahren nach einem der Ansprüche 1 bis 3, das derart ausgeführt ist, dass sich eine UV-Aushärtewellenlänge, die beim Aushärten des Silikonmaterials zum Einsatz kommt, von einer UV-Sterilisierungswellenlänge, die im Rahmen eines Sterilisierungsschritts des Silikonmaterials zum Einsatz kommt, unterscheidet.

11. Verwendung nach Anspruch 10, wobei das UV-Aushärten des Silikonmaterials in ausgehärtetem Zustand eine Härte im Bereich von Shore A40 bis Shore A60 aufweist.

## Claims

1. Method for connecting two silicone tube sections (1, 2) with following steps:
sterilizing the end face portions (1a, 2a) of the two silicone tube sections (1, 2) to be connected using a UV light irradiation with a UV sterilization wavelength;
positioning the end face portions (1a, 2a) of the silicone tube sections (1, 2) to be connected that face each other in an overmolding mold (13) such that the end face portions (1a, 2a) facing each other abut each other;
overmolding the end face portions (1a, 2a) abutting each other in the overmolding mold (13) by filling an overmolding cavity (14) of the overmolding mold (13) with flowable, UV curing silicone (15);
curing (21) the flowable silicone (15) by UV light irradiation using a UV light irradiation with a UV curing wavelength;
wherein the UV curing wavelength differs from the UV sterilization wavelength.

2. Pharmaceutical media transfer method for supplying a pharmaceutical medium (5) from a source reservoir (3) to a target reservoir (4) with following steps:
providing the pharmaceutical medium (5) in the source reservoir (3) including a silicone supply tube (6) being in media communication with the source reservoir (3);
providing the target reservoir (4) including a silicone discharge tube (7) being in media communication with the target reservoir (4);
displacing (9) the pharmaceutical medium (5) in a silicone supply tube section of the silicone supply tube (6);
cutting (10) the silicone supply tube section of the silicone supply tube (6) for creating a sterile end face portion (1a) of the silicone supply tube section;
cutting a silicone discharge tube section of the silicone discharge tube (7) for creating a sterile end face portion (2a) of the silicone discharge tube section;
connecting the silicone supply tube section with the silicone discharge tube section with a method according to claim 1, wherein the silicone supply tube section on the one hand and the silicone discharge tube section on the other hand represent the two silicone tube sections to be connected by the method;
transferring the pharmaceutical medium (5) from the source reservoir (3) to the target reservoir (4) via the connected tube sections (1, 2).

3. Pharmaceutical media transfer method according to claim 2, **characterized in that** the cutting (10) of the silicone supply tube section and/or the silicone discharge tube section is performed at a cutting temperature that is smaller than 80° C.

4. Apparatus (22) for connecting two silicone tube sections (1, 2) and for performing a method according to one of claims 1 to 3
with a UV source for sterilizing the end face portions (1a, 2a) of the two silicone tube sections (1, 2) to be connected using a UV light irradiation with a UV sterilization wavelength;
with a cutting unit (11) for cutting at least one of the silicone tube sections (1, 2),
with an overmolding mold (13) including a feeding device (16, 17) for the flowable silicone (15) as overmolding material,
with a positioning device (11a) for positioning the silicone tube sections (1, 2) to be connected; and
with a UV source for curing the flowable silicone (15) by UV light irradiation using a UV light irradiation with a UV curing wavelength;
wherein the UV curing wavelength differs from the UV sterilization wavelength.

5. Apparatus according to claim 4,
wherein the apparatus comprises a first UV source for sterilizing the end face portions (1a, 2a) of the two silicone tube sections (1, 2) to be connected using a UV light irradiation with a UV sterilization wavelength, and a second UV source for curing the flowable silicone (15) by means of UV light irradiation with a UV curing wavelength.

6. Apparatus according to claim 4,
wherein the apparatus comprises a collective UV source which is configured such that the respective irradiation wavelengths for sterilizing the end face portions (1a, 2a) of the two silicone tube sections (1, 2) to be connected and for curing the flowable silicone (15) can be set via corresponding filters.

7. Apparatus according to any of claims 4 to 6,
**characterized by** a mobile configuration for the laboratory use, the clinical use, the use in a clean room environment, or the use in a pre-series environment;
wherein the apparatus is configured to be mobile and comprises several castors, which are mounted on a frame of the apparatus;
wherein the apparatus in particular comprises a traction drive, especially an electric motor.

8. Apparatus according to any of claims 4 to 7, further comprising a set of blades (11); and
a set of overmolding molds (13);
wherein the tubes for the tube sections (1, 2) to be connected, namely the silicone supply tube (6) and the silicone discharge tube (7), have predefined data;
wherein the apparatus further comprises a reading unit (26) for presetting a blade within the set of blades (11) and/or for presetting an overmolding mold within the set of overmolding molds (13), each adapted to the tube sections (1, 2) to be connected.

9. Apparatus according to claim 8,
wherein the blades (11) of the set of blades (11) and the overmolding molds (13) of the set of overmolding molds (13) each have a code, in particular a QR code;
wherein the predefined data of the tubes (6, 7) for the tube sections (1, 2) to be connected are in the form of codes, in particular QR codes; and
wherein the reading unit (26) is configured as a code reader, in particular as a QR code reader.

10. Use of a liquid, UV curing silicone material (15) as overmolding material in a method according to one of the claims 1 to 3, which is configured such that a UV curing wavelength used in the curing of the silicone material differs from a UV sterilization wavelength used in a sterilization step of the silicone material..

11. Use according to claim 10, wherein the UV curing of the silicone material in cured state has a hardness in the range of Shore A40 to Shore A60.

## Revendications

1. Procédé pour connecter deux parties de tuyau en silicone (1, 2) comprenant les étapes suivantes :
stérilisation des zones frontales (1a, 2a) des parties de tuyau en silicone (1, 2) à connecter au moyen d'un rayonnement UV d'une longueur d'onde de stérilisation UV ;
positionnement des zones frontales (1a, 2a) tournées l'une vers l'autre des parties de tuyau en silicone (1, 2) à connecter dans un moule de surmoulage (13) de sorte que les zones frontales (1a, 2a) tournées l'une vers l'autre soient en contact l'une avec l'autre ;
surmoulage des zones frontales adjacentes (1a, 2a) dans le moule de surmoulage (13) par remplissage d'une cavité de surmoulage (14) du moule de surmoulage (13) avec une silicone fluide durcissant aux UV (15) ;
durcissement (21) de la silicone fluide (15) par irradiation aux UV au moyen d'un rayonnement UV d'une longueur d'onde de durcissement UV ;
la longueur d'onde de durcissement UV étant différente de la longueur d'onde de stérilisation UV.

2. Procédé de transfert de milieu pharmaceutique permettant d'acheminer un milieu pharmaceutique (5) d'un réservoir de départ (3) vers un réservoir de destination (4), comprenant les étapes suivantes :
mise à disposition du milieu pharmaceutique (5) dans le réservoir de départ (3), y compris un tuyau d'alimentation en silicone (6) relié au milieu du réservoir de départ (3) ;
mise à disposition du réservoir de destination (4), y compris un tuyau d'évacuation en silicone (7) relié au milieu du réservoir de destination (4) ;
refoulement (9) du milieu pharmaceutique (5) dans une partie du tuyau d'alimentation en silicone (6) ;
sectionnement (10) de la partie du tuyau d'alimentation en silicone (6) afin de créer une zone frontale stérile (1a) de la partie du tuyau d'alimentation en silicone ;
sectionnement d'une partie du tuyau d'évacuation en silicone (7) afin de créer une zone frontale stérile (2a) de la partie du tuyau d'évacuation en silicone ;
connexion de la partie de tuyau d'alimentation en silicone à la partie de tuyau d'évacuation en silicone à l'aide d'un procédé selon la revendication 1,
la partie de tuyau d'alimentation en silicone d'une part et la partie de tuyau d'évacuation en silicone d'autre part représentant les deux parties de tuyau en silicone à connecter à l'aide dudit procédé ;
transfert du milieu pharmaceutique (5) du réservoir de départ (3) vers le réservoir de destination (4) via les parties de tuyau (1, 2) connectées.

3. Le procédé de transfert de milieu pharmaceutique selon la revendication 2, **caractérisé en ce que** le sectionnement (10) de la partie du tuyau d'alimentation en silicone et/ou de la partie du tuyau d'évacuation en silicone se fait à une température de sectionnement qui est inférieure à 80 °C.

4. Dispositif (22) pour la connexion de deux parties de tuyau en silicone (1, 2) et pour la mise en œuvre d'un procédé selon l'une quelconque des revendications 1 à 3
avec une source d'UV destinée à stériliser les zones. frontales (1a, 2a) des parties de tuyau en silicone (1, 2) à connecter au moyen d'un rayonnement UV d'une longueur d'onde de stérilisation UV ;
avec une unité de sectionnement (11) permettant de sectionner au moins une des parties de tuyau en silicone (1, 2) ;
avec un moule de surmoulage (13) comprenant un dispositif d'alimentation (16, 17) pour la silicone fluide (15) en tant que matériau de surmoulage ;
avec un dispositif de positionnement (11a) permettant de positionner les parties de tuyau en silicone (1, 2) à connecter ; et
avec une source d'UV destinée à durcir la silicone fluide (15) par irradiation aux UV au moyen d'un rayonnement UV d'une longueur d'onde de durcissement UV ;
la longueur d'onde de durcissement UV étant différente de la longueur d'onde de stérilisation UV.

5. Le dispositif selon la revendication 4,
dans lequel le dispositif présente une première source d'UV pour stériliser les zones frontales (1a, 2a) des parties de tuyau en silicone (1, 2) à connecter au moyen d'un rayonnement UV d'une longueur d'onde de stérilisation UV et une deuxième source d'UV pour durcir la silicone fluide (15) au moyen d'un rayonnement UV d'une longueur d'onde de durcissement UV.

6. Le dispositif selon la revendication 4,
dans lequel le dispositif présente une source d'UV commune, réalisée de telle sorte que, grâce à des filtres appropriés, les longueurs d'onde d'irradiation respectives peuvent être prédéfinies pour stériliser les zones frontales (1a, 2a) des parties de tuyau en silicone (1, 2) à connecter et pour durcir la silicone fluide (15).

7. Le dispositif selon l'une quelconque des revendications 4 à 6, **caractérisé par** une conception mobile pour une utilisation en laboratoire, en clinique, dans un environnement de salle blanche ou dans un environnement de pré-série ;
le dispositif étant mobile et comportant plusieurs roulettes montées sur un châssis du dispositif ;
le dispositif comportant notamment un entraînement, en particulier un moteur électrique.

8. Le dispositif selon l'une quelconque des revendications 4 à 7, présentant en outre un ensemble de lames (11) ; et
un ensemble de moules de surmoulage (13) ;
dans lequel les tuyaux pour les parties de tuyau à connecter, à savoir le tuyau d'alimentation en silicone (6) et le tuyau d'évacuation en silicone (7), comportent des données prédéfinies ;
le dispositif présentant en outre une unité de lecture (26) permettant de spécifier une lame dans l'ensemble de lames (11) et/ou de spécifier un moule de surmoulage dans l'ensemble de moules de surmoulage (13), chacun étant adapté aux parties de tuyau à connecter.

9. Le dispositif selon la revendication 8,
dans lequel les lames (11) de l'ensemble de lames (11) et les moules de surmoulage (13) de l'ensemble de moules de surmoulage (13) comportent chacun un code, en particulier un code QR ;
dans lequel les données prédéfinies des tuyaux (6, 7) sont disponibles sous forme de codes, en particulier de codes QR, pour les parties de tuyau à connecter ; et
dans lequel l'unité de lecture (26) est réalisée sous forme de lecteur de code, en particulier de lecteur de code QR.

10. Utilisation d'un matériau en silicone liquide durcissant aux UV (15) comme matériau de surmoulage dans un procédé selon l'une quelconque des revendications 1 à 3, qui est réalisé de telle sorte qu'une longueur d'onde de durcissement UV utilisée pour durcir le matériau en silicone diffère d'une longueur d'onde de stérilisation UV utilisée dans le cadre d'une étape de stérilisation du matériau en silicone.

11. Utilisation selon la revendication 10, dans laquelle le durcissement UV du matériau en silicone présente, à l'état durci, une dureté comprise entre Shore A40 et Shore A60.
